# EUROPEAN PATENT APPLICATION

(11) **EP 1 695 655 A1**
(43) Date of publication of application: **30.08.2006**
(21) Application number: 04807213.6
(22) Date of filing: 10.12.2004
(51) Int. Cl.: A61B 1/00, A61M 25/00, G02B 23/24

(54) **ACTIVE TUBE AND ACTIVE TUBE SYSTEM**

(30) Priority: 12.12.2003 JP 2003415402
(71) Applicant: Japan Science and Technology Agency, Kawaguchi-shi, Saitama 332-0012 (JP); ESASHI, Masayoshi, Sendai-shi, Miyagi 982-0807 (JP); HAGA, Yoichi, Sendai-shi, Miyagi 9800803 (JP)
(72) Inventor: ESASHI, Masayoshi, Sendai-shi, Miyagi 9820807 (JP); HAGA, Yoichi, Sendai-shi, Miyagi 9800803 (JP); MIZUSHIMA, Masanori, Sendai-shi, Miyagi 9820807 (JP); MATSUNAGA, Tadao, Sendai-shi, Miyagi 9810952 (JP)
(74) Representative: Marshall, John Grahame
(86) International application number: PCT/JP2004/018855
(87) International publication number: WO 2005/055818

(57) **Abstract**

An active tube and its system are offered which can be directed by bending its tip, controlling the degree of bend, thereby easily capable of insertion into difficult positions, and which can be driven at low temperature at which it can be used for inspection and medical treatment.

A bending mechanism (21) is constructed by wiring an SMA coil (21e) along an outer side of a working channel tube (21a). The bending mechanism (21) is inserted into an outer skin tube (25) with a plurality of built-in weights (22). A front tip (23) is attached to the front end side of the bending mechanism (21) as a tip (2) of an active tube (1). A main tube (4) is connected through the working channel tube (21a) at the tip (2). A wire (21g) is connected to the SMA coil (21e). Said wire (21g) is inserted to the behind end side (41) of the main tube (4) in a wiring channel (4B9 of the main tube, thereby makes it possible to drive the bending mechanism (21) from outside.

## Description

### Technical Field

The present invention relates to an active tube and an active tube system conducting inspection or maintenance by entering complex machines or pipes, and further capable of utilization for diagnosis or medical treatment by entering as an active catheter into human blood vessels or organisms.

### Background Art

In recent years, an active endoscope which utilizes shape memory alloy as an actuator has been proposed for diagnosis of a large intestine and others, and an active catheter (narrow tubular tool) conducting inspection, diagnosis, or medical treatment by entering such a narrow space as blood vessels has been developed.
For example, various methods are proposed in a medical field so that a tube can be passed into a pylorus or a small intestine for so-called non-operational ileus treatment without celiotomy operation. As an example, there is a medical tool with an endoscope holding a tip of a tube, a tube with a guide wire as a core inserted therein, or a preliminarily inserted and left long overtube. Also, a tube which has a tip with a weight incorporated therein is widely used.

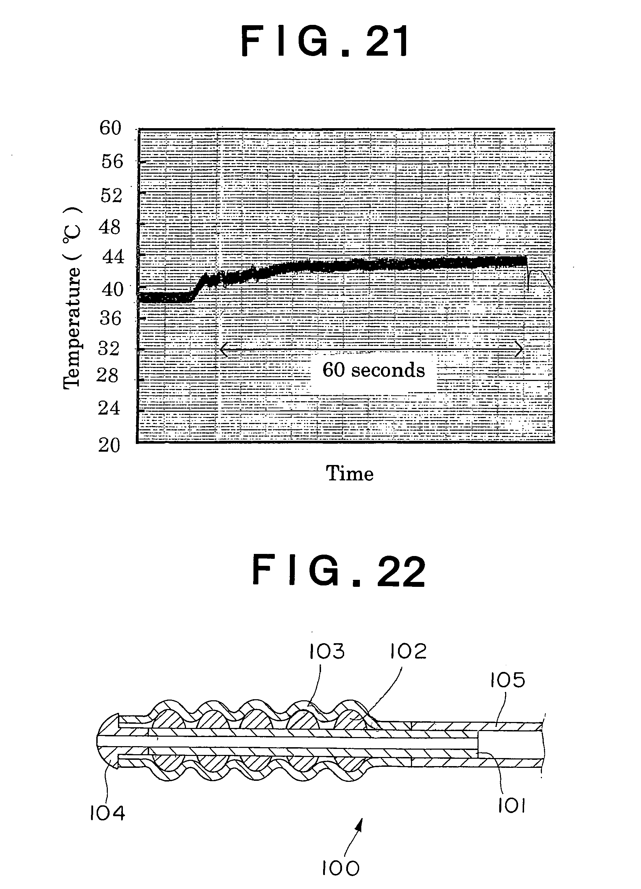

Fig. 22 is a cross-sectional view diagrammatically illustrating a tip of a conventional catheter with a weight incorporated at the tip. A conventional catheter 100 for ileus treatment is provided with circular weights 102 at pre-determined intervals in the longitudinal direction around a working channel tube 101 at a tip. And the outer peripheries of a plurality of weights 102 are covered with an outer skin tube 103. A tip 104 is provided at a tip of the catheter 100. On the other hand, the behind of the working channel tube 101 is connected to a void main tube 105.

The above-mentioned catheter 100 is used as follows. First, the catheter 100 is inserted through a nasal or mouth cavity. An operator decides the direction for a tip of the catheter 100 to proceed under the X-ray observation, and push ahead the catheter 100. In order to insert the catheter 100, the gravity acting on the weight 102 at the tip of the catheter 100 is utilized, that is, the tip of the catheter 100 is directed by changing the position of the patient variously. In some cases, the tip of the catheter 100 is directed to push with a hand from outside the body by the operator. There is also a method of insertion by harmonizing with the patient's breath.

Further in recent years, an active bending tube is developed which uses shape memory alloys (hereinafter, to be written "SMA"). For example, an active bending tube provided with a MIF (Multi-function Integrated Film) tactile sensor which can be inserted into a blood vessel was reported as "Development of a Microfine Active Bending Catheter equipped with MIF Tactile sensors" by Hironobu Takizawa, and four co-authors, IEEE International MEMS' 99 Conference, January 17, 1999. In said active bending tube, an SMA wire is buried in a tubule part provided within an outer skin tube, and the tip part is bent by applying a pulse wave of an amplitude of 160V with 20% duty cycle to the SMA wire for driving. In this case, the outer skin temperature by heating the SMA coil was about 80°C.

However, in order to insert a catheter 100 of conventional examples, there are many cases where the insertion is extremely difficult due to branching or narrowing of organs, so that it takes long time till inserting to a target part. There is also a problem that a heavy load is upon both an operator and a patient because the position of the patient in agonizing state must be changed variously. Moreover, there is also such a problem that, since the operator changes the patient's position according to his experience, it takes time to adjust the position to the inserting location if the inserting location of the catheter 100 is extremely difficult.

Also, since organs at or around the tip of the catheter 100 are observed under X-ray fluoroscopy, the patient and the operator are exposed long time to X-ray, which is not desirable for health. Moreover, there is also such a problem that, since the operator can not observe directly the inserting location of the catheter, its insertion is not easy.
There is also a problem that, though the active bending tube of Non-Patent Reference 1 can bend its tip, the condition required for medical instruments used inside a body to be 41°C or lower is not satisfied.

### Disclosure of the Invention

The object of the present invention is to offer an active tube and its system which can direct the tip by bending, easily insert to the difficult location by controlling the degree of bending, and can be driven at low temperature allowed for use for inspection and medical treatment.

In order to solve the above-described problems, the active tube in accordance with the present invention is characterized that it comprises; a working channel tube with its inside used as a working channel; an SMA coil aligned along said working channel tube; one or more of weights attached on the outer surface of the unified working channel tube and said SMA coil; and an outer skin tube covering the outer surface of said weights including said working channel tube and said SMA coil. By this constitution, it is possible to bend by driving the SMA coil of the active tube, to direct the active tube, to improve the insertion ability to difficult locations, and even in case that the SMA coil does not operate, it can be used as before.

Also, the active tube in accordance with the present invention is characterized that it comprises a tip and a main tube connected to said tip, and said tip comprises; a working channel tube connected through to said main tube; a bending mechanism to support and bend said working channel tube; one or more of weights attached to the outer surface of said bending mechanism; and an outer skin tube to cover the outer surface of said bending mechanism together with said weights, wherein said bending mechanism includes an SMA coil arranged in the longitudinal direction of said working channel tube. By this constitution, it is possible to direct the active tube by driving the bending mechanism at the tip of the active tube and bending it arbitrarily in the angle and the direction, and to improve the insertion ability to difficult locations. Since also weights are built in, it can be used as before even in case that the bending mechanism does not operate.

It is preferable that on the front end side of said main tube, a cylindrical thin film covers inflatable the outer surface of said main tube; and said main tube is provided with a balloon inflating channel along the axis of said main tube to supply gas or liquid into the space between said main tube and said thin film, thereby said thin film is inflated to form a balloon. By this constitution, the balloon can be inflated by sending such gas as air or such liquid as water and physiological saline into the channel for inflating a balloon from behind the main tube. Hereby, for example, it is possible to insert an active tube through a nasal or a mouth cavity of a human body to inflate the balloon at the pre-determined location. Thus, the balloon is contacted to the intestine wall by inserting the tip of the active tube into the intestine and inflating the balloon, thereby the active tube can be easily moved forward by peristaltic motion of the intestine.

Preferably, an endoscope is inserted into said working channel tube of said tip. By this constitution, the location where the active tube is inserted can be easily observed.
Preferably, said endoscope is built in said tip. By this constitution, since said endoscope is incorporated into said active tube, the diameter of the active tube itself can be made smaller.

According to another embodiment of the present invention, the active tube comprising a tip and a main tube connected to said tip is provided with an endoscope, and said tip is provided with:a working channel tube connected through to said main tube; a bending mechanism to support said working channel tube and to bend said working channel tube; and an outer skin tube covering the outer surface of said bending mechanism, said bending mechanism is provided with an SMA coil arranged in the longitudinal direction of said working channel tube, wherein a cylindrical thin film covers inflatable the outer surface of said main tube at the tip of said main tube, and said main tube is provided with a balloon inflating channel along the axis of said main tube to supply air or liquid into the space between said main tube and said thin film, thereby said thin film is inflated to form a balloon. By this constitution, the insertion ability to difficult locations is improved by driving the bending mechanism at the tip of the active tube and bending it arbitrarily in the angle and the direction, thereby directing the active tube. Also, the balloon can be inflated by sending such gas as air or such liquid as water and physiological saline into the channel for inflating the balloon from behind the main tube. Hereby, for example, it is possible to insert an active tube through a nasal or a mouth cavity of a human body to inflate the balloon at the pre-determined location, thereby the balloon is contacted to the intestine wall by inserting the tip of the active tube into the intestine and inflating the balloon. Thus, the active tube can be easily moved forward by peristaltic motion of the intestine. Further, if said active tube is provided with the endoscope, the forward area around the balloon can be easily observed by inserting the tip of the active tube into the intestine, inflating the balloon, and by bending the tip of the endoscope inserted into the working tube with the balloon as a so-called supporting point.

Preferably, at the tip of said endoscope are provided an image input part comprising an optical fiber or an image pickup device, and a light guide for illumination or LED to illuminate the forward of said image input part. By this constitution, the forward of the active tube is illuminated, the illuminated position is input via the image input part, and thereby images can be taken out.
Preferably, said bending mechanism is provided with a pair of links attached at an interval to said working channel tube; and an outer skin contacted to said pair of links and covering said working channel tube; wherein an air layer is formed with said pair of links and the outer surface of said working channel tube, and said SMA coil is inserted through each small diameter hole of said pair of links to be wired to said air layer. By this constitution, the heat is generated by the electrical current to the SMA coil, but the heat is easily dissipated via the air layer. Also, the bending mechanism is made up with a working channel tube, an outer skin, and others, and since the proportion of space is high, it is easily bent, and thereby only a small amount of electrical current to the SMA coil is required. Also together with the formation of air layer between the outer skin of the bending mechanism and the outer skin tube, the heat does not tend to be accumulated at the tip so as to lower the temperature rise.

Preferably, said SMA coil is inserted through a first small diameter hole of a behind link and a first small diameter hole of a front link, bent back at the front end of said front link, inserted through a second small diameter hole of said front link and a second small diameter hole of said behind link, and is wired. By this constitution, the front and the behind of the SMA coil can be fixed with the link, and the SMA coil is contracted by the electrical current therethrough owing to the shape memory alloy effect, and thereby bending motion of the tip can be attained.
Preferably, said SMA coil is inserted through each small diameter hole of the behind link and each small diameter hole of the front link between said pair of links, bent back a plurality of times, and is wired. Hereby, the bending strength of the bending mechanism can be improved.

Preferably, a plurality of said SMA coils are provided at equal intervals with respect to the central axis of said working channel tube between said pair of links. Hereby, the bending directions by respective SMA coils provided as the bending mechanism become mutually different, and the bending directions by respective SMA coils are mutually related to make the tip bend in multi-directions.
Preferably, said main tube is provided along the axis of said main tube with a working channel connected through to said working channel tube and a wiring channel to insert the wire to be connected to the SMA coil of said bending mechanism. By this constitution, the wiring for electrical current to the SMA coil can be introduced from the behind end of the main tube, and various medicines and things can be inserted if necessary from the behind end of the main tube through the working channel.

An active tube system in accordance with the present invention is characterized to comprise said active tube, a control box to control the bending mechanism of said active tube, and a control input part to input the control information of the bending mechanism to the control box. By this constitution, the bending mechanism of the active tube can be driven via the control input part.
Preferably, said control input part has a control stick with a formed grip and said control stick is provided with a slide type operational mechanism which can be grabbed with a palm. Hereby, the control information can be easily input with respect to the bending mechanism of the active tube.

### Brief Description of the Drawings

Fig. 1 is a diagrammatical view illustrating the configuration of an active tube system as the best form of embodiment of the present invention.
Fig. 2 (a) is an exploded perspective view illustrating the configuration of a tip of the active tube, and (b) is a cross-sectional view of the tip of the active tube.
Fig. 3 (a) is a partial perspective view of a bending mechanism, and (b) is a cross-sectional view of the bending mechanism.
Fig. 4 is a cross-sectional view along the line A - A of Fig. 3(b).
Fig. 5 is a partial cross-sectional view of a behind part of a main tube.
Fig. 6 is a cross-sectional view along the line B - B of Fig. 1.
Fig. 7 is a diagrammatical cross-sectional view illustrating the structural part of a balloon attached at the tip side of the main tube.
Fig. 8 is a diagrammatical side view illustrating the structural part of a balloon attached at the tip side of the main tube.
Fig. 9 is a block functional diagram of a control box.
Fig. 10 is a cross-sectional view illustrating the bending state of the tip of the active tube.
Fig. 11 is a diagrammatical view of an active tube system in accordance with the second embodiment.
Fig. 12 is a diagrammatical diagonal view illustrating an example of another endoscope different from that of Fig.11.
Fig. 13 is a diagrammatical view of an active tube in accordance with the third embodiment.
Fig. 14 is a diagrammatical diagonal view illustrating an example of an endoscope.
Fig. 15 is a diagrammatical view illustrating the first embodiment of using the active tube system.
Fig. 16 is a diagrammatical view illustrating the second embodiment of using the active tube system.
Fig. 17 is a graph showing a bending angle of the tip with respect to the duty ratio of pulse voltage.
Fig. 18 is a graph showing the surface temperature change with time at the tip of the active tube in the atmosphere of 26°C.
Fig. 19 is a graph showing the surface temperature change with time at the tip bent in bending angle 30° in the soaked state into water of 38°C.
Fig. 20 is a graph showing the surface temperature change with time at the tip bent in bending angle 45° in the soaked state into water of 38°C.
Fig. 21 is a graph showing the surface temperature change with time at the tip bent in bending angle 60° in the soaked state into water of 38°C.
Fig. 22 is a diagrammatical cross-sectional view of the tip of the catheter as the background art of the present invention.

### Best Modes for Carrying out the Invention

Hereinafter, the present invention will better be understood with reference to the drawing figures attached hereto illustrating the present invention in detail and several embodiments of the present invention. Here, the various examples illustrated in the attached drawings are by no means intended to specify or limit the present invention, but only to facilitate explanation and understanding thereof.
Hereinafter, the best modes for carrying out the present invention will be described with reference to the drawing figures. In the description below, the case is explained where an active tube is an active tube for medical treatment, in particular, an ileus tube (long intestinal tube) used for treatment of ileus, but it is not necessarily limited to an active tube for medical treatment.

### [First embodiment]

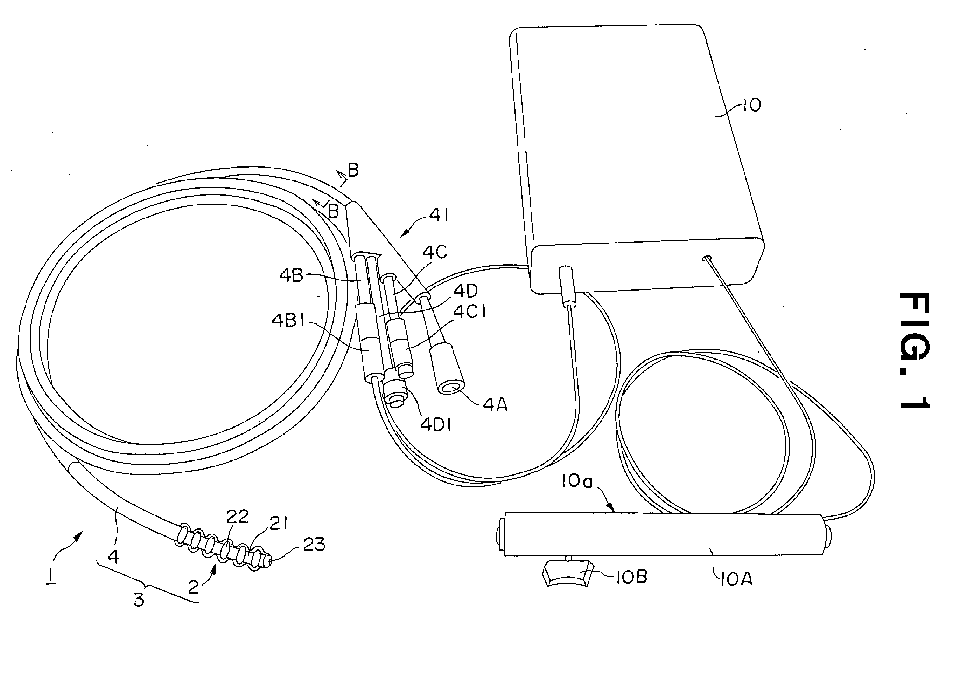

Fig. 1 is a diagrammatical view illustrating the whole configuration of an active tube system as the best form of embodiment of the present invention. An active tube system 1 comprises an active tube 3 provided with a tip 2 having a built-in bending mechanism 21, and a control box 10 to control the bending mechanism 21 of the active tube 3. A control input part 10a such as a manually controllable stick is externally connected to the control box 10.
The active tube 3 comprises a tip 2 with the built-in bending mechanism 21 and a plurality of circular weights 22 attached thereto, and a main tube 4 connected to the behind end of the tip 2.
A working channel 4A attached to a central axis of the main tube 4 aslant backward used as an intra-intestine sucking tube to suck the content accumulated inside an intestine such as a small intestine, a channel for wiring 4B, a channel for balloon inflating 4C to send air or liquid into a balloon attached at the tip of the main tube 4, and a channel for bent 4D to introduce to a ventilating hole around the tip of the main tube 4 are connected to the behind end 41 of the main tube 4.

### First of all, the active tube 3 is explained.

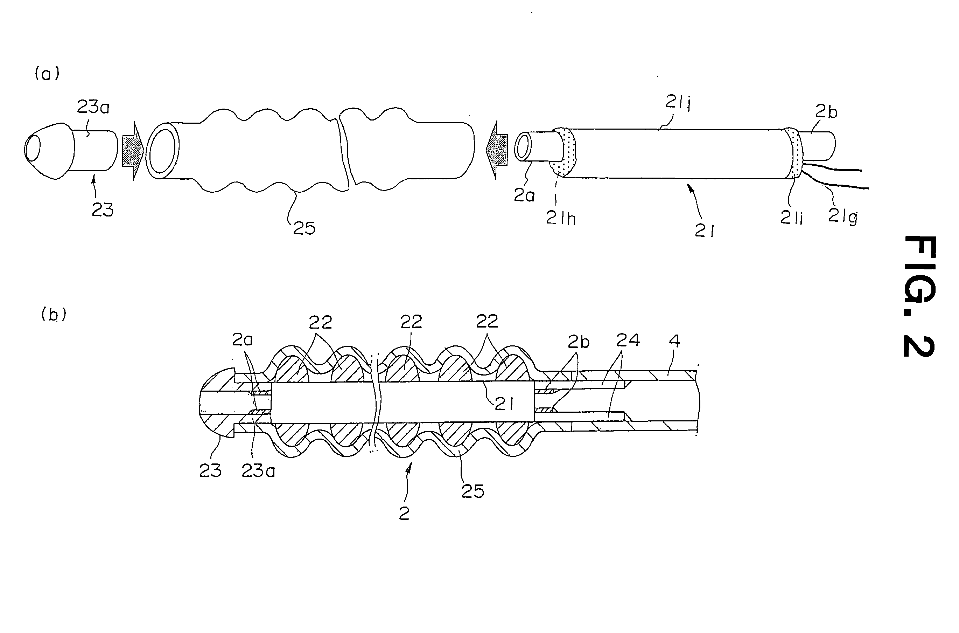

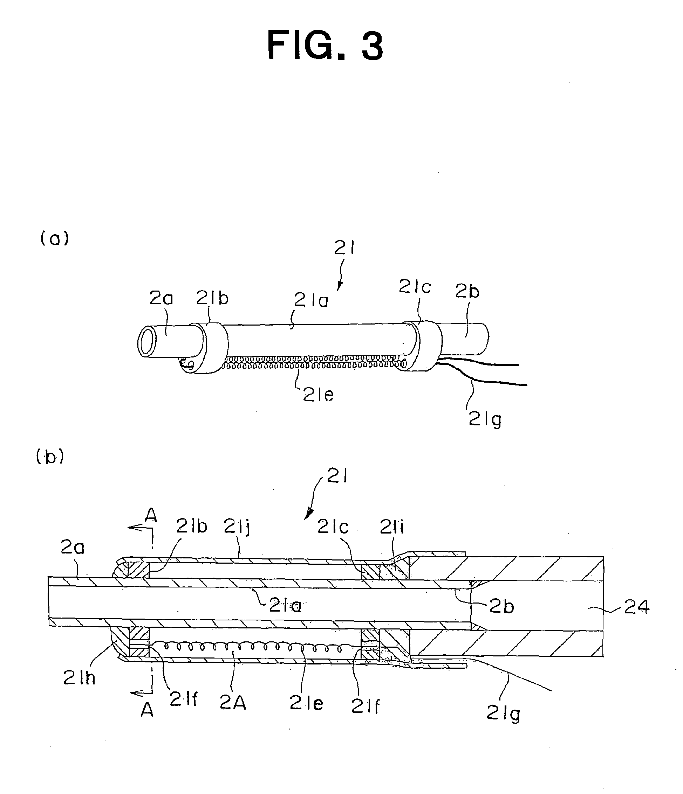

Fig. 2 is a view illustrating the structure of the active tube, and Fig. 2 (a) is an exploded perspective view illustrating the configuration of a tip of the active tube 3, and (b) is a cross-sectional view of the tip of the active tube 3. A tip part 2 with a built-in bending mechanism 21 in the longitudinal direction of the tube is provided at the tip side of the active tube 3. As shown in Fig. 2(a), the tip 2 of the active tube 3 is made up so as to insert the bending mechanism 21 from behind an outer skin tube 25 with circular weights 22 built in at a plurality of constrictions, and to be attached with a tip 23 from the front.
Here, as explained by Fig. 3(a) and (b) described below, a working channel tube 21a is inserted through a pre-determined hole of the bending mechanism 21.

At the front end of the tip 2, a working channel tube 21a is extended to form a front connection flange 2a. The outer surface of the front connection flange 2a is connected to a behind connection flange 23a of the tip 23. Said tip 23 forms a penetration hole of the same diameter as the cylindrical behind connection flange 23a, and has a smooth shape at the front end.
Also at the behind end of the tip 2, the working channel tube 21a is extended to form a behind connection flange 2b. At the outer surface of said behind connection flange 2b is connected a joint tube 24, and at the outer surface of the joint tube 24 is connected a main tube 4. Hereby, the tip 2 is connected to the main tube 4.

As shown in Fig. 2(b), a plurality of circular weights 22 are embedded at pre-determined intervals on the outer surface of the bending mechanism 21. That is, said weights 22 are attached to said outer surface together with said working channel tube 21a and an SMA coil constituting the bending mechanism 21. Further on the outer surface side of the bending mechanism 21, an outer skin tube 25 is partially attached over a plurality of circular weights 22. The front side of the outer skin tube 25 is connected to the outer surface of a behind side connection flange 23a of a tip 23, and the behind side of the outer skin tube 25 is connected to the outer surface of a joint tube 24. Said circular weights 22 is made of, for example, stainless steel, and, for example, a thin wall silicone tube can be used as the outer skin tube 25.

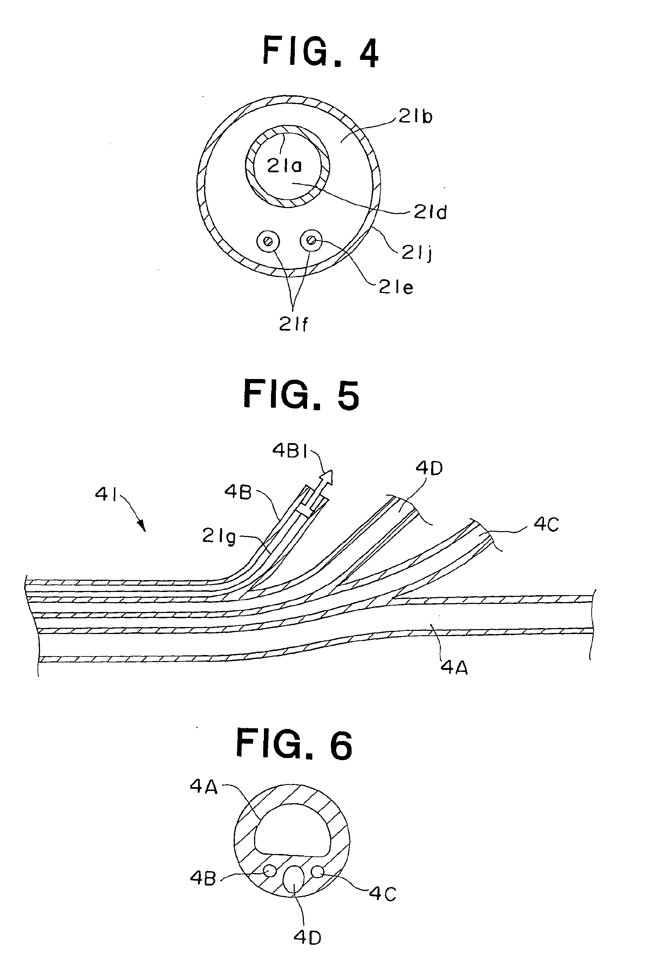

Explanation is next made of an example of the bending mechanism 21. Figs. 3 and 4 are views illustrating the structure of the bending mechanism.
In Fig. 3, (a) is a partial perspective view of the bending mechanism 21, and (b) is a cross-sectional view of the bending mechanism 21. As shown in Fig. 3(a), a working channel tube 21a is supported on a front link 21b and a behind link 21c. That is, the front end of the working channel tube 21a is inserted through a large diameter hole 21d of the front link 21b to form a front end connection flange 2a at the inserted front end. The behind end of the working channel tube 21a is inserted through a large diameter hole 21d of the behind link 21c, and the inserted behind end forms a behind end connection flange 2b and is connected to a joint tube 24.

As shown in Fig. 3(b), an SMA coil 21e formed in a spiral shape is inserted through a first small diameter hole 21f of the behind link 21c, and is wired with flexure if necessary to the first small diameter hole 21f of the front link 21b. The SMA coil 21e is inserted through the first small diameter hole 21f of the front link 21b and bent back, and is inserted through a second small diameter hole 21f of the front link 21b and through the second small diameter hole 21f of an opposite behind link 21c. The two ends of the SMA coil 21e are connected with solder and others to a wire 21g made of another material, for example, insulated copper wire. Here, the front link 21b and the behind link 21c are of the identical shape, and can be formed with a UV-sensitive resin which is hardened by UV irradiation by, for example, stereo lithography.
The working channel tube 21a is covered with an insulative outer skin 21j. The both ends of the outer skin 21j are fixed to the outer surface of the front link 21b and the behind link 21c. Further, the behind part of the outer skin 21j covers the front end side outer surface of the joint tube 21a so as to contact the adhesive 21i which support and fix the behind link 21c and the working channel tube 21a.

Fig. 4 is a cross-sectional view along the line A - A of Fig. 3(b). As is illustrated, a large diameter hole 21d penetrating the working channel tube 21a and two small diameter holes 21f penetrating the SMA coil 21e made of SMA are made in the front link 21b and the behind link 21c.
In said bending mechanism 21, the space 2A as shown in the figure in the longitudinal direction of SMA coil 21e is surrounded with the front link 21b and the behind link 21c. That is, the space 2A is an air layer. Owing to it, the outer skin 21j, the working channel tube 21a, and the outer skin tube 25 are made flexible, and thereby the tip 2 has a structure which easily dissipates heat and the heat can not be easily accumulated therein. It is further preferable to use a material with low phase transformation temperature for the SMA coil 21e. For example, the phase transformation temperature may be 60°C. Hereby, the generated heat can be reduced, and the temperature rise of the bending mechanism 21 by electrical current can be suppressed by providing an air layer for thermal conductivity in the space 2A around the SMA coil 21e.

Here, the bending mechanism 21 illustrated in Figs. 3 and 4 has such a structure that one SMA coil 21e is bent back only once at the front link 21b, but the wiring method of the SMA coil 21e is not limited to this manner. For example, one SMA coil 21e may be bent back several times for wiring at the front link 21b and the behind link 21c. That is, the SMA coil 21e may be inserted through respective small diameter holes made in the front link 21b and the behind link 21c and bent back several times. By this constitution, the tip 2 can be made flexible. In this case, both ends of one SMA coil 21e are preferably made to come out on the side of the main tube 4 of the behind link 21c.

Furthermore, a plurality of SMA coils 21e may be wired symmetrically. That is, on the cross-sectional plane (the cross-sectional plane on A - A line of Fig. 3(b)) with the longitudinal direction of the bending mechanism 21 as the normal line direction, each SMA coil 21e may be wired so as to be mutually symmetrical with respect to the central axis of the bending mechanism 21. In case to wire three SMA coils 21e, for example, they may be wired so the respective SMA coils 21e make 120°C mutually with respect to the central axis of the bending mechanism 21. In this case, the tip 2 can be bent in any direction by mutually associating the bending directions of respective SMA coils 21e. Here, each SMA coils 21e may be bent back only once or several times between the front and the behind links 21b and 21c.

Explanation is next made of a main tube 4. Fig. 5 is a partial cross-sectional view of a behind part 41 of a main tube 4, and Fig. 6 is a cross-sectional view along the line B - B of Fig.1. The main tube 4 is provided at least with a working channel 4A and a wiring channel 4B with the inserted wire 21g over the longitudinal direction. Also, depending upon the purpose of use, for example, in the first embodiment, a multi-channel type forming one or several tubes may well do as other channels in addition to a working channel 4A and a wiring channel 4B.

As shown in Fig. 6, in case that the active tube 3 is an ileus treatment active tube, most of the main tubes 4 are occupied with a working channel 4A of the pre-determined shape, and a bent channel 4D with the diameter smaller than the working channel 4A is formed between the working channel 4A and the outer surface of the main tube 4, and a wiring channel 4B with the diameter smaller than the bent channel 4D and a balloon inflating channel 4C are formed at both ends of the bent channel 4D. Here, the main tube 4 is formed with a flexible material that may be preferably, for example, silicone.

Here, each of the behind ends 41 of the wiring channel 4B, the balloon inflating channel 4C, and the bent channel 4D is equipped with each specific connector 4B1, 4C1, and 4D1, respectively. The end of the working channel 4A may also be equipped with a lid, a connector, or others. For example, as the connector 4B1 of the wiring channel 4B with the inserted wire 21g, a monoral jack corresponding to a pair of wires 21g or a stereo jack corresponding to two pairs of wires 21g may be mentioned.

Explanation is next made of a balloon attached at the tip side of the main tube 4.

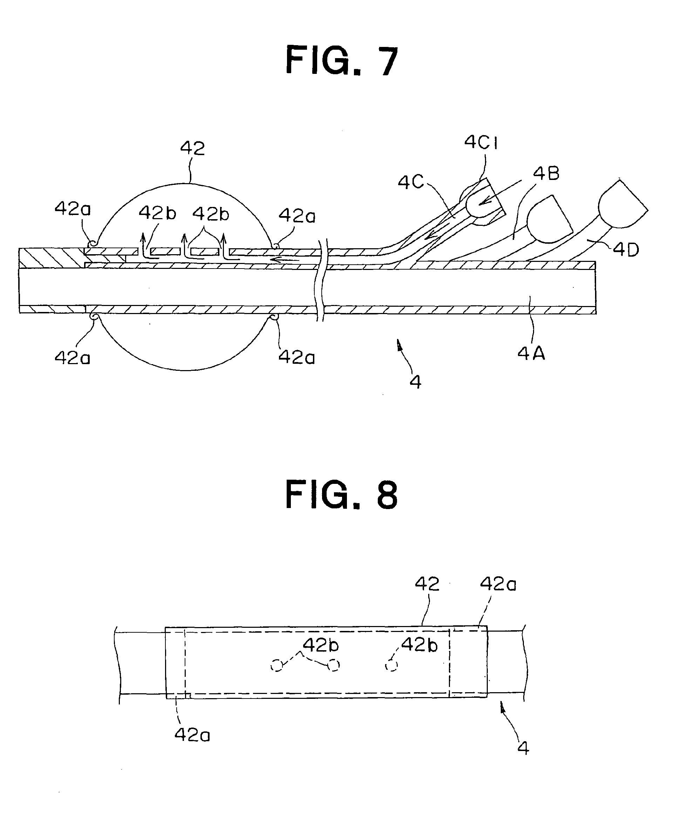

Fig. 7 is a diagrammatical cross-sectional view illustrating the structural part of a balloon attached at the tip side of the main tube 4, and Fig. 8 is a side view of said balloon. In the pre-determined position at the tip side of the main tube 4, a balloon 42, for example, made of elastic thin film is attached. Both front and behind parts of the balloon 42 are fixed to the outer surface of the main tube 4 with an adhesive 42a or the like. In the main tube 4, three injection holes 42b, for example, are made in the position covered with the balloon 42 (See Fig. 8.). Hereby, a gas such as air or a liquid such as water passed through the balloon inflating channel 4C passes through the injection holes 42b to inflate the balloon 42, as shown with an arrow mark in Fig. 7. Here, the cylindrical thin film used for the balloon 42 may be made of silicone rubber for example.

### A manufacturing method of the active tube 3 is explained below.

A bending mechanism 21 of a tip 2 is assembled first. That is, as shown in Fig. 3(a), the front tip side of the working channel tube 21a is inserted through the large diameter hole 21d of the front link 21b so as to extend. Also, the behind tip side of the working channel tube 21a is inserted through the large diameter hole 21d of the behind link 21c so as to extend. The front and the behind links 21b and 21c are tentatively adhered to the outer surface of the working channel tube 21a with an adhesive or others if necessary. Also, the SMA coil 21e is made of SMA. As the SMA coil 21e, for example, that of the component wire diameter 50 - 100 µm and the outer diameter 200 - 300 µm is used. One end of the SMA coil 21e is inserted through the first small diameter hole 21f of the behind link 21c and through the first small diameter hole 21f of the front link 21b. Thereafter, the tip of the SMA coil 21e inserted through the first small diameter hole 21f of the front link 21b is bent back, inserted through the second small diameter hole 21f of the front link 21b, and then is inserted through the second small diameter hole 21f of the behind link 21c. Both ends of the SMA coil 21e, that is, both ends towards the behind link 21c are connected to a different wire if necessary.

The outer skin 21j covers the working channel tube 21a in the longitudinal direction, the outer surface of the front link 21b and the inner surface of the front end side of the outer skin 21j are adhered, and an insulative adhesive 21h is piled up on the front side face of the front link 21b. The working channel tube 21a, the front link 21b, the SMA coil 21e, and the outer skin 21j are tightly adhered with an adhesive 21h. Similarly, the outer surface of the behind link 21c and the inner surface of the behind end side of the outer skin 21j are adhered, and the insulative adhesive 21i is piled up on the behind side face of the behind link 21c. The working channel tube 21a, the behind link 21c, the SMA coil 21e (or a wire 21g), and the outer skin 21j are tightly adhered with the adhesive 21i. As the adhesives 21h and 21i, silicone, for example, can be used.

Next, the joint tube 24 is connected to the outer side of the behind end connection flange 2b of the bending mechanism 21, and the behind end connection flange 2b and the inner surface of the front side of the joint tube 24 are fixed with an insulative adhesive, resulting in a bending mechanism 21.
Next, as shown in Fig. 2(a), the assembled bending mechanism 21 is inserted from behind the outer skin tube 25, and the front tip 23 is attached to the bending mechanism 21. That is, the inner surface of the behind end connection flange 23a of the front tip 23 and the end face of the front end connection flange 2a of the working channel tube 21a are adhered with an insulative adhesive. Hereby, the tip 2 of the active tube 3 is manufactured. The size of the tip 2 is, for example, several mm outer diameter and several tens of mm length.

Both ends of the SMA coil 21e of the tip 2 of the active tube 3 are connected with solder or others to the wire 21g inserted through the wiring channel 4B of the main tube 4. Here, in case that another wire is connected to both ends of the SMA coil 21e if necessary, said another wire is connected to the wire 21g. The active tube 3 is completed by connecting through the main tube 4 and the tip 2, and adhering the behind end face of the joint tube 24 and the inner surface of the front side of the main tube 4 with an insulative adhesive.

### Explanation is next made of a control box 10.

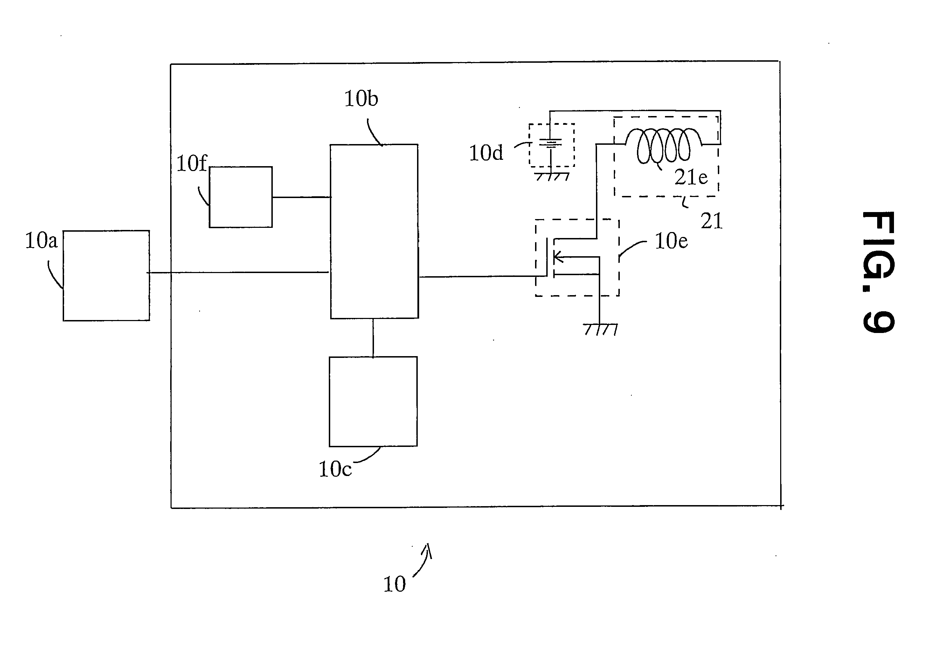

Fig. 9 is a block functional diagram of a control box 10. The control box 10 controls the bending mechanism 21 built in the tip 2 of the active tube 3. By said control, the tip 2 is bent. For example, as shown in Fig. 9, the control box 10 is provided with a control input part 10a comprising a control stick with a built-in potentiometer and others, a control part 10b to receive the input signals from the control input part 10a and to output the control signals to the bending mechanism 21, and a power source part 10c to supply electricity to the control part 10b. Here, said control input part 10a is a control stick which is a stick 10A having a formed grip so to be easily grabbed with a palm, and is provided with a slide type operational mechanism capable of input by manually moving a lever 10B. The control part 10b is constituted with a microcomputer and others, and the power source part 10c is constituted with a dry cell and others.

The electrical current to the SMA coil 21e from a direct current source 10d connected to its one end is controlled by a control device 10e. A dry cell can be used as said direct current source 10d, and a power transistor can be used as said control device 10e.
Here, instead of the control input part 10a of the control box 10, an input part 10f built in the control box 10 may be used.
Especially as the control system for the bending mechanism 21, a pulse width modulation (PWM) system can be used. By adopting the PWM system, the conducting time and the non-conducting time to the SMA coil 21e is repeated, thereby heating time and heat-dissipating time by the electrical current to the SMA coil 21e are repeated. By this, the temperature rise of the SMA coil 21e is suppressed, and thereby the temperature of the bending mechanism 21 and the tip 2 itself can be suppressed. Also the bending angle of the bending mechanism 21 can be controlled by changing the duty ratio of the conducting time, thereby change the current in one cycle.

The active tube system 1 thus constituted operates as is following.

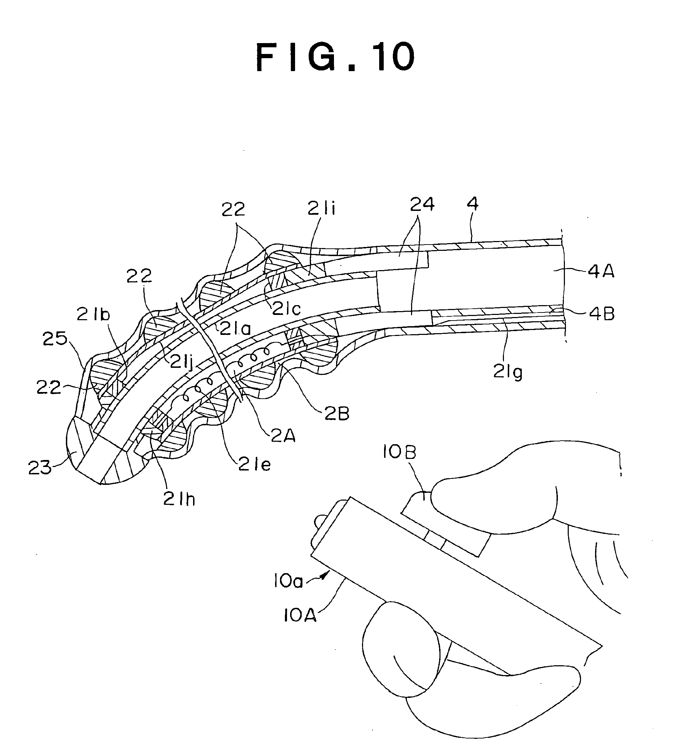

Fig. 10 is a cross-sectional view illustrating the bending state of the tip 2 of the active tube 3 when a lever 10B of a control stick as the control input part 10a of the control box 10 is operated. The lever 10B of the control stick is operated to input to the control part 10b of the control box 10, and the control device 10e is controlled according to said input. The control device 10e changes the current of one cycle to the SMA coil 21e. Hereby, the bending mechanism 21 functions, and the bending angle of the tip 2 is held constant, corresponding to the current to the SMA coil 21e.

Here, the SMA coil 21e heats up by the electrical current, but, as shown in Fig. 10, the bending mechanism 21 has a space 2A surrounded with the working channel tube 21a, the outer skin 21j, and the front and the behind links 21b and 21c on the front and the behind filled in with air, and further a space 2B between the outer skin 21j and the outer skin tube 25 is also filled in with air, that is, it has the air layers comprising the spaces 2A and 2B. Therefore, it has the structure in which the inside of the tip 2 has spaces 2A and 2B, and the outer skin 21j, the working channel tube 21a, and the outer skin tube 25 are made flexible, thereby the tip 2 is easily heat dissipated. Also, a material with low phase transformation temperature, for example, 60°C is used for the working channel tube 21a. Hereby, the generated heat itself is suppressed, and temperature rise of the bending mechanism 21 by the current to the SMA coil 21e can be suppressed by heat dissipation effect of the air layer.

### [Second Embodiment]

Explanation is next made of the active tube system in accordance with the second embodiment of the present invention.

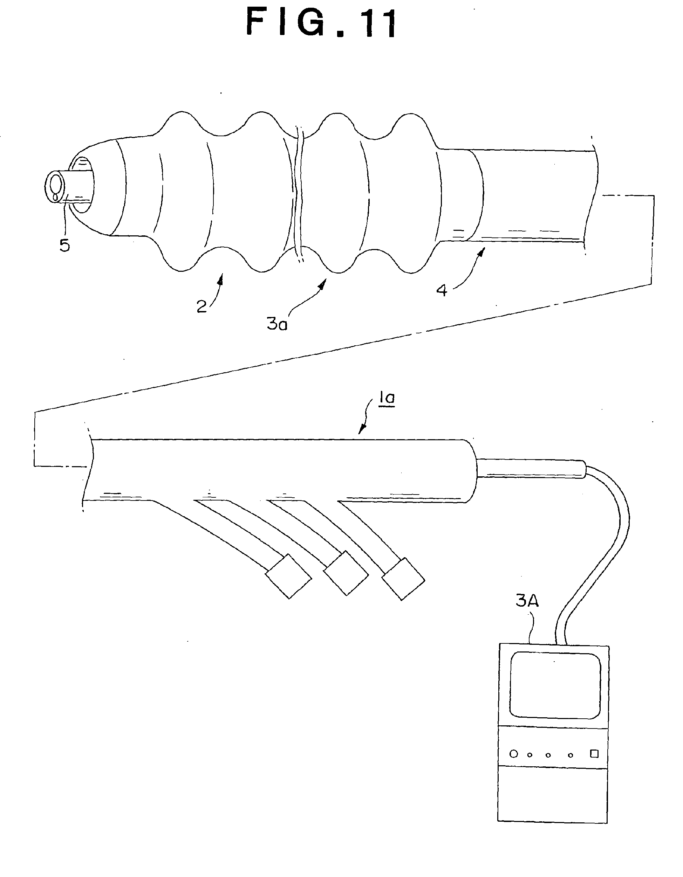

Fig. 11 is a diagrammatical view of an active tube system in accordance with the second embodiment. The active tube system 1a in accordance with the second embodiment comprises an active tube 3a and a display apparatus 3A. The active tube 3a is such that an endoscope 5 is inserted from a working channel 4A of the main tube 4 into the working channel tube 21a of the active tube 3 in accordance with the first embodiment. In said second embodiment, the front end side of the endoscope 5 is not fixed at the tip 2 of the active tube 3a. That is, the endoscope 5 is capable of insertion and detachable to the tip 2 via the main tube 4.
Hereby, the endoscope 5 can be inserted into the tip 2 via the main tube 4, and only the endoscope 5 is taken out if necessary, thereby a medicine can be inserted, and the content can be sucked out from the tip 2. Also, the endoscope 5 can be re-utilized if necessary after treating such as cleaning.

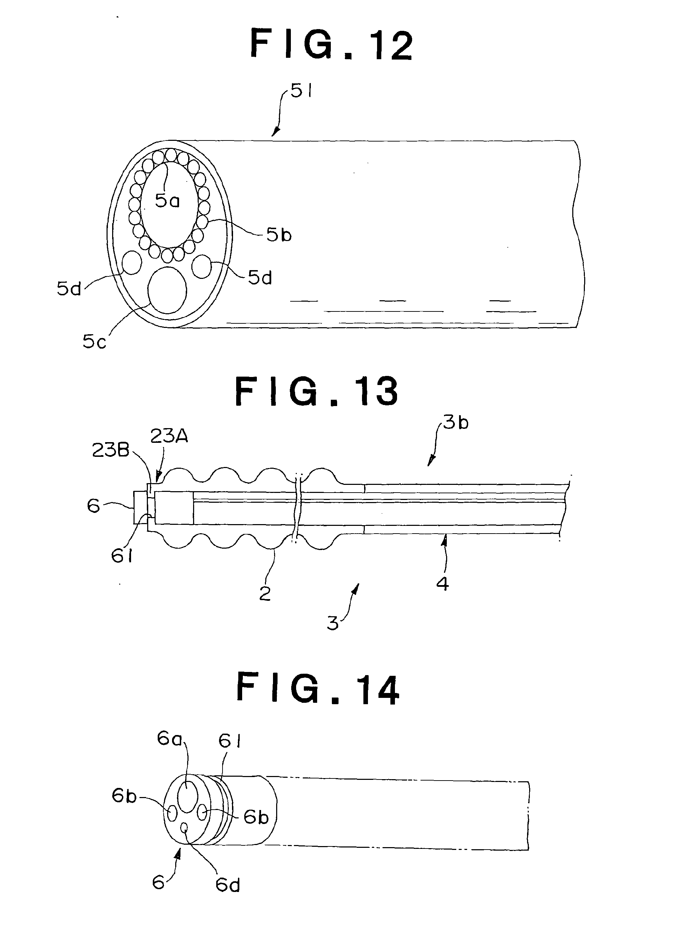

Fig. 12 is a diagrammatical perspective view illustrating an example of another endoscope different from that 5 of Fig. 11. The front side face of the endoscope 51 shown in Fig. 12 is provided with an image input part 5a comprising such an image pickup device such as an optical fiber and a charge coupled device (CCD), and a plurality of light guides 5b for illuminating forward of said image input part 5a. Also, the endoscope 51 may be provided with working channel 5c. The endoscope 51 may be also provided with an introduction tube to introduce physiological saline to clean the display plane of the image input part 5a, and the front side face of the endoscope 51 may be provided with an injection nozzle as the tip end of the induction tube. Here, instead of the light guide for illumination 5b, a light emitting diode (LED) may be used.

### [Third Embodiment]

Explanation is next made of the active tube system in accordance with the third embodiment of the present invention.
Fig. 13 is a diagrammatical view of an active tube in accordance with the third embodiment. An active tube 3b in accordance with the third embodiment is such that an endoscope 6 is inserted through the working channel tube 21a of the active tube 3 in accordance with the first embodiment, and the front end of said endoscope 6 is fixed to a tip 23A of the active tube 3b. On the inner surface of the tip 23A, there is a hooking part to hook the endoscope 6. Said hooking part is formed, for example, by enxtending a collar 23B to the axis side of the tip 23A. The hooking part of the tip 23A is hooked with the hooking part of the endoscope 6, for example, a concave part 61 so that the endoscope 6 is not detached from the tip 23A.

Fig. 14 is a diagrammatical perspective view illustrating an example of an endoscope 6. The front side face of the endoscope 6 is provided with an image input part 6a comprising an image pick-up device such as a CCD, and a plurality of LED 6b to illuminating the forward of the image input part 6a. The endoscope 6 may also be provided with a channel for different operation (not shown). An introduction tube to introduce physiological saline may be provided for cleaning a display plane of the image input part 6a, and an injection nozzle 6d may be provided to the front side face of the endoscope 6 as the front end of the introduction tube.

Here, the image input part 6a may not be the CCD but may output images to outside using optical fiber as in the second embodiment. It may also have a light guide for illumination instead of a LED 6b as in the second embodiment, and a light guide for illumination may guide the external light to illuminate in front of the image input part 6a. Here, the hooking parts are provided to the endoscope 6 and a front tip 23A to fix the endoscope 6 to the front tip 23A, but it may be fixed with adhesive or others.
As the endoscope in the above-mentioned second and third embodiments, for example, those with the diameter of 3 - 10 mm, or those of ultra-thin, for example, of 2.5 mm may be mentioned.

Hereinafter, an explanation is made of the method of use of the active tube system. Here, the explanation is specifically made with an ileus treatment in mind.

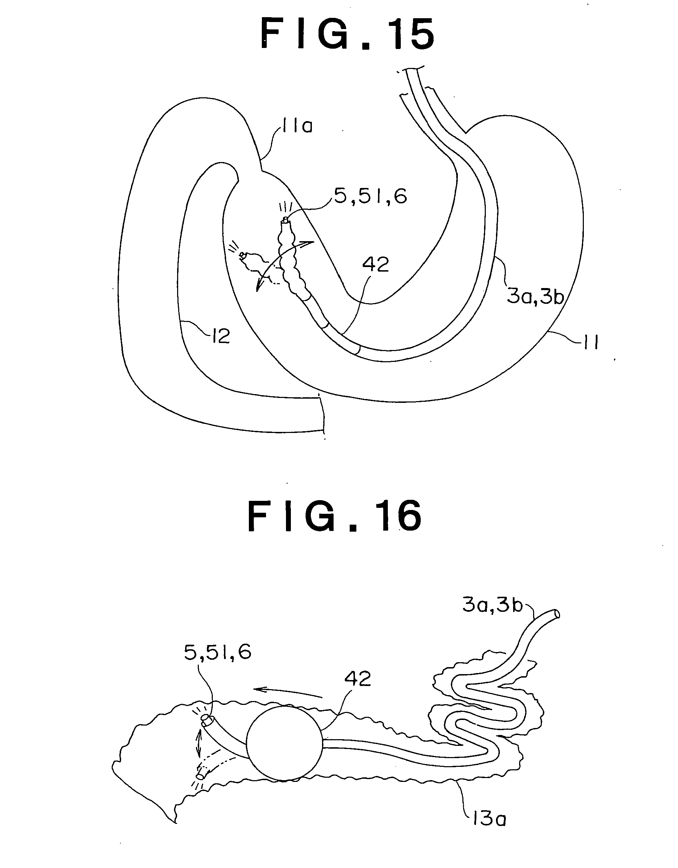

Fig. 15 is a diagrammatical view illustrating the first embodiment of using the active tube system. In the figure, the case in which active tubes 3a and 3b are inserted through a nasal or a mouth cavity to proceed in the body and pass a pylorus part 11a as the outlet of a stomach 11 is shown. Said pylorus part 11a is so small that it is a position difficult to insert. In case of the active tubes 3a and 3b with the inserted endoscope, the operator makes observation of the images from the image input parts 5a and 6a at the tip of the endoscopes 5, 51, and 6 by the display apparatus 3A and others to operate the control input part 10a of the control box 10. By the operation of the control input part 10a, the tip 2 of the active tube 3 is bent.
For example, by moving the lever 10B of the control stick, the bending angle of the tip 2 of the active tube 3 can be arbitrarily changed as shown with an arrow mark in the figure. By bending the tip 2 of the active tube 3, the tip 2 of the active tubes 3a and 3b are driven toward the pylorus part 11a, and easily inserted into an intestine duodenum 12.

Fig. 16 is a diagrammatical view illustrating the second embodiment of using the active tube system. The figure illustrates the state of the active tubes 3a and 3b inserted through the stomach 11 further into the intestine. Behind the tips 2 of the active tubes 3a and 3b, the balloon 42 attached at the front side of a main tube 4 is inflated, and made to contact to the intestine wall 13a. By contacting said balloon 42 to the intestine wall 13a, the front sides of the active tubes 3a and 3b are fixed.

In this state, as explained in the first method of use, the operator makes the observation of the images from the image input parts 5a and 6a at the tip of the endoscopes 5, 51, and 6 by a display apparatus 3A and others to operate the control input part 10a of the control box 10. Thereby, the tips 2 of the active tubes 3a and 3b are bent by the operation of the control input part 10a. For example, by moving the lever 10B of the control stick, the bending angle of the tip 2 of the active tube 3 can be arbitrarily changed as shown with an arrow mark. Thereby, the operator can observe the intestine wall 13a at will. Also with the intestinal peristaltic motion, the balloon 42 can be moved forward in the intestine.

Thus, since the active tubes 3a and 3b connect the main tube 4 with the attached balloon 42 and the tip 2 with a built-in bending mechanism 21, as well as include the image input parts 5a and 6a comprising such image pickup devices as the optical fiber and CCD at the tip 2, they can be used as an endoscope to observe inside the intestine.
Here, in the first and the second methods of use, the explanation is made on the premise of the active tubes 3a and 3b with an inserted endoscope, but the active tube 3 without an inserted endoscope can be similarly used as well by utilizing together X-ray transmission images.

Also, in the first through the third embodiments, the bending mechanism 21 at the tip 2 comprises the SMA coil 21e, but the tip 2 can be bent in all directions by symmetrically arranging a plurality of SMA coils 21e on the cross-sectional plane of the bending mechanism 21, and heating by electrical current to each SMA coil 21e. In this case, the control input part 10a can be realized by, for example, providing slide type control mechanisms of the same number as SMA coils 21e to the control stick.

### [Fourth Embodiment]

Explanation is next made of a fourth embodiment referring to Fig. 16.
The fourth embodiment is such that the plurality of weights 22 are not provided at tips 2 of the active tube 3a of the second embodiment or of the active tube 3b of the third embodiment. That is, the active tube 3 comprises the tip 2 with a built-in bending mechanism 21 and the main tube 4 connected to the behind end of the tip 2, provided with endoscopes 5, 51, and 6 inserted into the working channel 4A of the main tube 4 and the working channel tube 21a at the tip 2. The constitutions of the bending mechanism 21 and the main tube 4 are same as in other embodiments.

### [Example]

### An active catheter for ileus was prepared as below.

The SMA coil 21e of the outer diameter 200 - 300 µ m was prepared using the titanium- nickel (Ti-Ni) SMA of the component wire having a diameter of 50 - 100 µm, and a bending mechanism 21 was assembled therewith, and a tip 2 was prepared. Here, the length of the tip 2 was about 40mm, and the outer diameter was 6mm. The tip 2 was connected to a main tube 4. The bending mechanism 21 was driven by the pulse of peak voltage of 16.5V and its period of 86.2Hz, and the tip 2 was bent by change of the duty ratio.

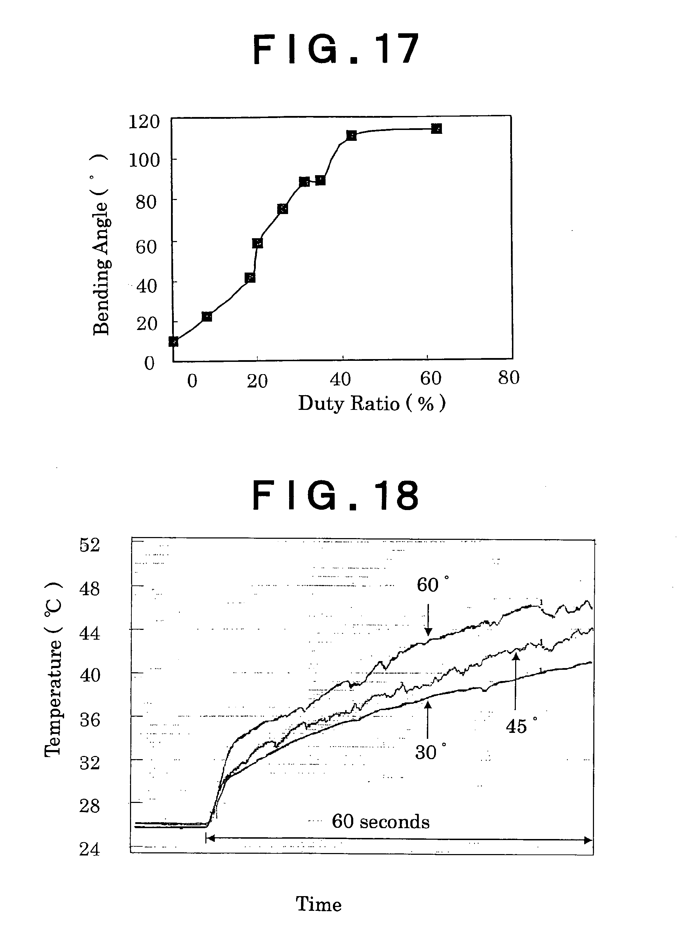

Fig. 17 is a graph showing a bending angle of the tip 2 with respect to the duty ratio of the pulsed voltage. The bending angle is the angle of the extended line of the main tube 4 and the tip 2. For each duty ratio, it took within one second for the bending mechanism 21 to operate and for the tip 2 to bend. Also, the tip 2 is positioned on the extended line of the main tube 4 by returning the lever 10B of the control stick to the original position.
As seen from Fig. 17, the bending motion was possible up to 110° at most by the duty ratio 40%, wherein the radius of curvature was about 20mm. It was also confirmed that the tip 2 could be bent with a guide wire of the diameter 1.14 mm inserted through the main tube 4 and the working channel tube 21a.

Fig. 18 is a graph showing the surface temperature change with time at the tip of the active tube 3 in the atmosphere of 26°C. The surface temperature of the outer skin tube 25 of the tip 2 was measured with a thermocouple by applying electrical current to the SMA coil 21e so as the bending angle to be 30° , 45° , and 60° with the duty ratio fixed to the pre-determined value. The thermocouple was set in the position where directly received the radiation heat from the SMA coil 21e, that is, more concretely, it was contacted to the outer skin tube 25 between weights 22. As is obvious from the figure, the times from the starting of current till when the surface temperature exceeded 41° were seen to be 60, 40, and 25 seconds, respectively, for the bending angles 30° , 45° , and 60° .
Further, the case being presumed where the active tube 3 was used as the active tube for ileus treatment, that is, the location of its use was inside a stomach or an intestine, and there was food residue and the like. The surface skin temperature of the active tube was confirmed in the state soaked in the water of 38°C.

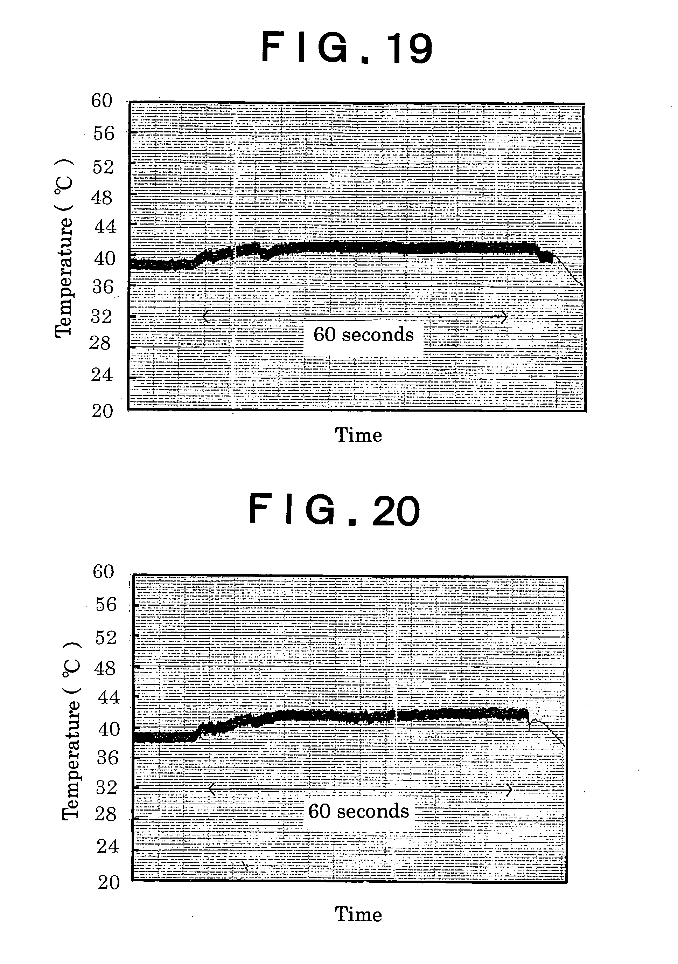

Figs. 19 - 21 are a graph showing the surface temperature change with time at the tip 2 bent in bending angles 30° ,45° , and 60° , respectively, in the soaked state into water of 38°C. As is seen from these figures, the surface temperature was constant at about 41°C at the bending angle 30° , and at about 42°C at the bending angle 45° . At the bending angle 60° , it did not rise to about 44°C even when the bent state was held for one minute or longer. It is assumed that it was due to the improvement of heat efficiency, because, on the outermost surface of the tip 2 of the active tube 3, there was water having the better thermal conductivity than that of the atmosphere. Therefore, it turned out that it is possible enough to use it for medical treatment, for, at the bending angle 30° , the surface temperature was about 41°C or lower.

The various embodiments described heretofore are mere examples to embody the present invention, and variations are possible within the scope of the description in the claims of the present invention, and it is needless to say that these are also to be included in the present invention.

### Industrial Applicability

The active tube in accordance with the present invention is such that the front end of the active tube is easily bent in an arbitrary direction and at an arbitrary angle, thereby directing the active tube is possible, and insertion is made easy into the location where it would otherwise be difficult. In case that the weights are built in the tip, the conventional way of using is also possible by the gravitational function of the weight. On the other hand, in case that an endoscope is provided, and a balloon is attached to a main tube, the inserted location can be observed while the active tube is being inserted.

Since, inside and around the bending mechanism, the structure is so made that heat is easily dissipated, the heat generated by electrical current to the SMA coil of the bending mechanism can be easily dissipated, thereby the temperature rise can be suppressed at the tip of the active tube.
It can also be used as, for example, a medical tool for observation inside an intestine. Further, the active tube system in accordance with the present invention is such that the bending mechanism of the active tube can be easily driven via the controller.

## Claims

1. An active tube, wherein it comprises;
a working channel tube the inside of which is used as a working channel;
an SMA coil arranged along said working channel tube;
one or more weights attached on the outer surface of the combined working channel tube and SMA coil; and
an outer skin tube covering the outer surface of said weight including said working channel tube and said SMA coil.

2. An active tube, wherein it comprises a tip and a main tube connected to said tip, and said tip is comprises:
a working channel tube connected through to said main tube;
a bending mechanism to support said working channel tube and to bend said working channel tube;
one or more weights attached on the outer surface of said bending mechanism; and
an outer skin tube covering the outer surface of said bending mechanism together with said weight, wherein
said bending mechanism includes an SMA coil arranged in the longitudinal direction of said working channel tube.

3. The active tube as set forth in Claim 2, wherein, on the front end side of said main tube, a cylindrical thin film covers inflatable the outer surface of said main tube, and
said main tube is provided with a balloon inflating channel along the axis of said main tube to supply gas or liquid into the space between said main tube and said thin film, thereby said thin film is inflated to form a balloon.

4. The active tube as set forth in Claim 2, wherein an endoscope is inserted into said working channel tube of said tip.

5. The active tube as set forth in Claim 2, wherein said endoscope is built in said tip.

6. An active tube, wherein it comprises a tip and a main tube connected to said tip, and it is provided with an endoscope; and
said tip is provided with:
a working channel tube connected through to said main tube;
a bending mechanism to support said working channel tube and to bend said working channel tube; and
an outer skin tube covering the outer surface of said bending mechanism,
said bending mechanism is provided with an SMA coil arranged in the longitudinal direction of said working channel tube,
wherein a cylindrical thin film covers inflatable the outer surface of said main tube at the tip of said main tube, and
said main tube is provided with a balloon inflating channel along the axis of said main tube to supply air or liquid into the space between said main tube and said thin film, thereby said thin film is inflated to form a balloon.

7. The active tube as set forth in any one of Claims 4-6,
wherein the front end of said endoscope is provided with an image input part comprising an optical fiber or an image pickup device and a light guide for illumination or LED to illuminate the forward of said image input part.

8. The active tube as set forth in Claim 2 or Claim 6, wherein;
said bending mechanism is provided with a pair of links attached at an interval to said working channel tube; and
an outer skin contacted to said pair of links and covering said working channel tube; and
an air layer is formed with said pair of links and the outer surface of said working channel tube, and
said SMA coil is inserted through each small diameter hole of said pair of links to be wired to said air layer.

9. The active tube as set forth in Claim 8, wherein said SMA coil is inserted through a first small diameter hole of a behind link and a first small diameter hole of a front link, bent back at the front end of said front link, inserted through a second small diameter hole of said front link and a second small diameter hole of said behind link, and is wired.

10. The active tube as set forth in Claim 8, wherein said SMA coil is inserted through each small diameter hole of the behind link and each small diameter hole of the front link between said pair of links, bent back a plurality of times, and is wired.

11. The active tube as set forth in Claim 8, wherein a plurality of said SMA coils are provided at equal intervals with respect to the central axis of said working channel tube between said pair of links.

12. The active tube as set forth in Claim 2 or Claim 6,
wherein; said main tube is provided along the axis of said main tube with a working channel connected through to said working channel tube and a wiring channel to insert the wire to be connected to the SMA coil of said bending mechanism.

13. An active tube system, wherein it comprises an active tube, a control box to control a bending mechanism of said active tube, and a control input part to input the control information for said bending mechanism to said control box; and
said active tube comprises a tip and a main tube connected to said tip; and
the tip of said active tube is provided with;
a working channel tube connected through to said main tube;
a bending mechanism to support said working channel tube and bend said working channel tube;
one or more weights attached to the outer surface of said bending mechanism; and
an outer skin tube covering the outer surface of said bending mechanism together with said weight; and
said bending mechanism includes an SMA coil arranged in the longitudinal direction of said working channel tube.

14. The active tube system as set forth in Claim 13, wherein, on the front end side of said main tube, a cylindrical thin film covers inflatable the outer surface of said main tube; and
said main tube is provided with a balloon inflating channel along the axis of said main tube to supply gas or liquid into the space between said main tube and said thin film, thereby said thin film is inflated to form a balloon.

15. The active tube system as set forth in Claim 13, wherein an endoscope is inserted into said working channel tube of said tip.

16. The active tube system as set forth in Claim 13, wherein said endoscope is built in said tip.

17. An active tube system, wherein it comprises an active tube, a control box to control a bending mechanism of said active tube, and a control input part to input the control information for said bending mechanism to said control box;
said active tube comprises a tip and a main tube connected to said tip; and said active tube is provided with an endoscope;
said tip is provided with a working channel tube connected through to said main tube, a bending mechanism to support said working channel tube and bend said working channel tube, and an outer skin tube covering the outer surface of said bending mechanism;
said bending mechanism includes an SMA coil arranged in the longitudinal direction of said working channel tube; and
on the front end side of said main tube, a cylindrical thin film covers inflatable the outer surface of said main tube; and
said main tube is provided with a balloon inflating channel along the axis of said main tube to supply air or liquid into the space between said main tube and said thin film, thereby said thin film is inflated to form a balloon.

18. The active tube system as set forth in any one of Claims 15 - 17, wherein the front end of said endoscope is provided with an image input part comprising an optical fiber or an image pickup device and a light guide or LED for illumination to illuminate the forward of said image input part.

19. The active tube system as set forth in Claim 13 or Claim 17, wherein;
said bending mechanism is provided with a pair of links attached at an interval to said working channel tube; and
an outer skin contacted to said pair of links and covering said working channel tube; and
an air layer is formed with said pair of links and the outer surface of said working channel tube; and
said SMA coil is inserted through each small diameter hole of said pair of links to be wired to said air layer.

20. The active tube system as set forth in Claim 19, wherein said SMA coil is inserted through a first small diameter hole of a behind link and a first small diameter hole of a front link, bent back at the front end of said front link, inserted through a second small diameter hole of said front link and a second small diameter hole of said behind link, and is wired.

21. The active tube system as set forth in Claim 19, wherein said SMA coil is inserted through each small diameter hole of the behind link and each small diameter hole of the front link between said pair of links, bent back a plurality of times, and is wired.

22. The active tube system as set forth in Claim 19, wherein a plurality of said SMA coils are provided at equal intervals with respect to the central axis of said working channel tube between said pair of links.

23. The active tube system as set forth in Claim 13 or Claim 17, wherein;
said main tube is provided along the axis of said main tube with;
a working channel connected through to said working channel tube; and
a wiring channel to insert the wire to be connected to the SMA coil of said bending mechanism.

24. The active tube system as set forth in Claim 13 or Claim 17, wherein; said control input part has a control stick with a formed grip and said control stick is provided with a slide type operational mechanism which can be grabbed with a palm.
